# EUROPEAN PATENT APPLICATION

(11) **EP 2 848 605 A1**
(43) Date of publication of application: **18.03.2015**
(21) Application number: 14184816.8
(22) Date of filing: 15.09.2014
(51) Int. Cl.: C07C 51/12, C07C 51/363, C07C 51/377, C07C 53/122, C07C 53/19, C07C 57/04

(54) **Methods for preparing acrylic acid from biobased starting materials**

(30) Priority: 16.09.2013 US 201361878493 P
(71) Applicant: Armstrong World Industries, Inc., Lancaster, Pennsylvania 17603 (US)
(72) Inventor: Lensbouer, Joshua, Mt. Joy, PA Pennsylvania 17552 (US)
(74) Representative: Jenkins, Peter David

(57) **Abstract**

Described herein is a method for preparing acrylic acid comprising the followings steps:
(a) providing a source of ethanol;
(b) carbonylating the ethanol in the presence of a metal catalyst to obtain propanoic acid;
(c) halogenating the propanoic acid to obtain halopropanoic acid;
(d) heating the halopropanoic acid in the presence of an inorganic base; and
(e) recovering acrylic acid.

## Description

### Cross-Reference to Related Application

This application claims the benefit of priority from U.S. Provisional Application Serial No. 61/878,493, filed 16 September 2013, the contents of which are hereby incorporated herein in their entirety.

### Field

Embodiments of the present invention provide methods for preparing acrylic acid from biobased starting materials.

### Background

Acrylic acid monomers are materials, which easily polymerize to form acrylic resins. Acrylic acids can also be esterified to form acrylic acid esters. Both acrylic acid and acrylic acid esters self-polymerize, and also combine with other co-monomers such as acrylamides, acrylonitrile, vinyl, styrene, and butadiene to create polymers, as needed for a particular end use. Acrylic polyacrylate homopolymers are used in the manufacture of superabsorbent polymers (SAPs), thickeners, detergents and dispersants, while acrylic heteropolymers are used to manufacture a broad range of plastics, coatings, adhesives, elastomers, polishes, and paints.

The dwindling supply of natural resources, the rising costs of petroleum, concerns over greenhouse gas emissions and the future availability of industrial chemicals, and consumer demand for more environmentally friendly products has sparked a desire to shift from production of chemicals derived from petroleum sources to chemicals derived from sustainable materials, particularly biobased materials. However, challenges exist in the development of biobased materials, including: 1) adequate supply of starting materials; 2) competitive production processes; and 3) industry acceptance of a reduced number of alternatives. Despite these challenges, there is a need and desire for commercially suitable biobased chemicals. Specifically, the UV/EB curable industry stands to benefit from the availability of such materials.

Moreover, conventional methods of preparing biobased industrial chemicals have required complex processes having multiple steps, which increases both the time and cost of obtaining such materials. For example, the production of biobased 1,3-propanediol - an organic compound which has numerous uses across multiple industries - requires at least the following process steps: microfiltration and ultrafiltration, ion exchange, flash evaporation, and distillation. Accordingly, there remains a need for efficient and cost-effective processes, which produce high yields of biobased industrial chemicals. Embodiments of the present invention are directed to these and other ends.

### Summary

In some embodiments, the present invention provides a simple, relatively high yield synthesis for acrylic acid comprising: heating ethanol in the presence of an alumina to obtain ethylene gas; hydroxycarbonylating the ethylene gas, optionally in the presence of a catalyst, to obtain propanoic acid; halogenating the propanoic acid to obtain a halopropanoic acid; and heating the halopropanoic acid in the presence of an inorganic base to obtain acrylic acid.

In some embodiments, the present invention provides a simple, relatively high yield synthesis for acrylic acid comprising: carbonylating ethanol in the presence of a catalyst, to obtain propanoic acid; halogenating the propanoic acid to obtain a halopropanoic acid; and heating the halopropanoic acid in the presence of an inorganic base to obtain acrylic acid.

### Detailed Description

In some embodiments, the present invention provides a simple, relatively high yield synthesis for acrylic acid comprising: heating ethanol in the presence of an inorganic acid to obtain ethylene; carbonylating the ethylene, optionally in the presence of a catalyst, to obtain propanoic acid; halogenating the propanoic acid to obtain halopropanoic acid; and heating the halopropanoic acid in the presence of an inorganic base to obtain acrylic acid.

In some embodiments, the propanoic acid may be halogenated with homonuclear diatomic halogen molecules selected from the group consisting of fluorine (F₂), chlorine +(Cl₂), bromine (Br₂), and iodine (I₂).

In some embodiments, the inorganic acid is selected from the group consisting of oxidizing acids: phosphoric acid, nitric acid, and sulfuric acid are a few. In some embodiments the ethanol is heated to a temperature of from about 40 °C to 120 °C. In some embodiments the ethanol is heated to a temperature of less than about 120 °C. In some embodiments, the ethanol is heated to about 70 °C.

In some embodiments, the hydroxycarbonylation of the ethylene is carried out in aqueous media. In some embodiments, the carboxylation of the ethylene is carried out in the presence of a catalyst. In some embodiments, the catalyst is a metal carbonyl catalyst. In some embodiments, the catalyst is a nickel carbonyl catalyst, or another metal carbonyl where the metal is from Group 4-12 on the periodic table.

In some embodiments, the present invention provides a simple, relatively high yield synthesis for acrylic acid comprising: carbonylating ethanol in the presence of a catalyst, to obtain propanoic acid; halogenating the propanoic acid to obtain halopropanoic acid; and heating the halopropanoic acid in the presence of an inorganic base to obtain acrylic acid.

In some embodiments, the methods of the present invention follow Scheme I described below.

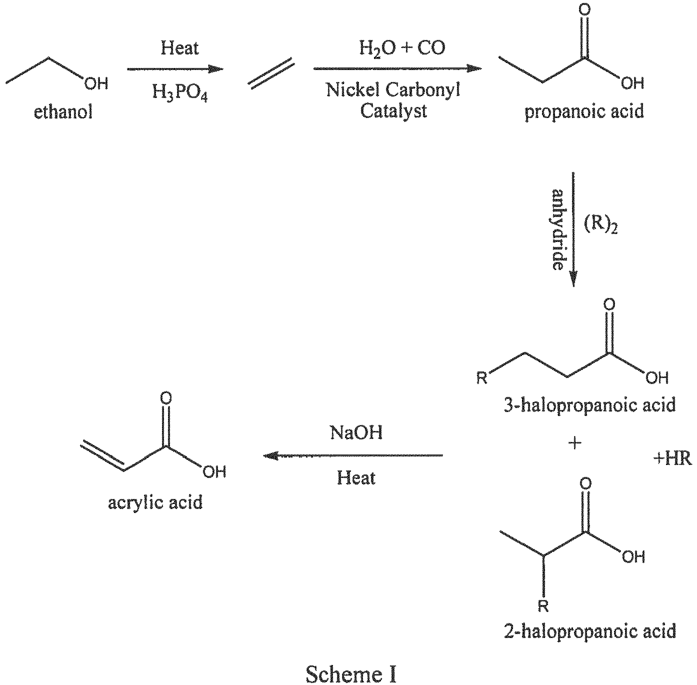

Wherein R is a halogen atom selected from the group consisting of fluorine, chlorine, bromine, and iodine.

In some embodiments, the present invention provides a simple, relatively high yield synthesis for acrylic acid comprising: carbonylating ethanol in the presence of a catalyst, to obtain propanoic acid; halogenating the propanoic acid to obtain halopropanoic acid; and heating the halopropanoic acid in the presence of an inorganic base to obtain acrylic acid.

In some embodiments, the ethanol is the product of a fermentation process.

In some embodiments, the ethanol is carbonylated in the presence of a metal carbonyl catalyst. In some embodiments, the ethanol is carbonylated in the presence of a nickel carbonyl catalyst.

In some embodiments, the method comprises treating propionic acid with between 80 and 90 percent of its molecular equivalent of halogen, in the presence of an anhydride (e.g. acetic anhydride) at a temperature between about 50 °C and about 140 °C while stirring the reacting mixture. In some embodiments, a slow feed of halogen is used ranging from 0.1-0.5 % weight of the total reaction weight of halogen per minute into the propanoic acid until 90 % of the total theoretical amount of halogen is added to make monohalopropanoic acid. Halopropanoic acid is then fractionally distilled and collected.

In some embodiments, the halopropanoic acid may include 2-fluoropropanoic acid, 3-fluoropropanoic acid, 2-chloropropanoic acid, 3-chloropropanoic acid, 2-bromopropanoic acid, 3-bromopropanoic acid, 2-iodopropanoic acid, 3-iodopropanoic acid, as well as mixtures thereof.

In some embodiments, the halopropanoic acid is a mixture of 2-chloropropanoic acid and 3-chloropropanoic acid. In some embodiments, the halopropanoic acid is 3-chloropropanoic acid.

In some embodiments, the halopropanoic acid is heated in the presence of an inorganic base to a temperature that does not exceed about 80 °C. In some embodiments, the halopropanoic acid is heated in the presence of an inorganic base to a temperature that does not exceed about 75 °C. In some embodiments, the inorganic base is sodium hydroxide or potassium hydroxide. In some embodiments, the inorganic base is potassium hydroxide.

In some embodiments, the methods of the present invention follow Scheme II described below.

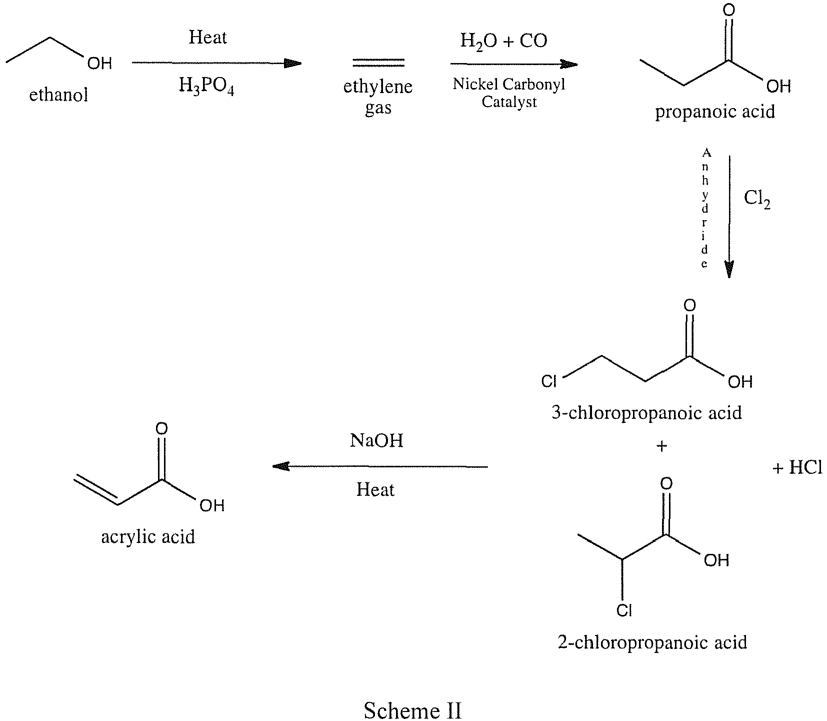

In some embodiments, the method further comprises the step of agitating the mixture of inorganic base and halopropanoic acid. In some embodiments, the mixture of inorganic base and halopropanoic acid is mixed for about 60 minutes.

In some embodiments, the method further comprises the addition of an inorganic acid to the mixture of halopropanoic acid and potassium hydroxide. In some embodiments, the mixture is a solution. In some embodiments, the inorganic acid is added in an amount sufficient to acidify the solution.

In some embodiments, the method further comprises heating the acidified solution. In some embodiments, the acidified solution is heated to about 100 °C. In some embodiments, the acidified solution is heated until water distills off.

In some embodiments the method further comprises the step of collecting the water that distills off.

Some embodiments further comprise the step of adding 4-methoxyphenol. In some embodiments, the 4-methoxyphenol inhibits polymerization.

Some embodiments further comprise the step of heating the solution to a temperature sufficient to distill off acrylic acid. Some embodiments further comprise the step of heating the solution to a temperature of about 140 °C. Some embodiments further comprise the step of recovering the acrylic acid.

Some embodiments further comprise the step of purifying the acrylic acid. In some embodiments, purification is carried out by vacuum distillation. In some embodiments the vacuum distillation decreases the temperature for distillation. In some embodiments, purification is carried out by solvent extraction followed by distillation.

In some embodiments, the salt slurry is mixed. In some embodiments, the mixing of the salt slurry improves the yield. In some embodiments, the yield is between 70 and 80%.

In some embodiments, the purity of the acrylic acid is at least 90%. In some embodiments the purity of the acrylic acid is between 90 and 95%. In some embodiments the purity of the acrylic acid is greater than 95%.

In some embodiments, the methods of the present invention comprise the following steps:
(a) providing a form of ethanol derived from a fermentation process;
(b) carbonylating the ethanol in the presence of a metal catalyst to obtain propanoic acid;
(c) halogenating the propanoic acid to obtain a mixture comprising 3-halopropanoic and 2-halopropanoic acid; and
(d) heating the mixture comprising 3-halopropanoic and 2-halopropanoic acid in the presence of an inorganic base to obtain acrylic acid.

In some embodiments, step (c) is carried out in the presence of an anhydride (e.g. acetic anhydride).

In some embodiments, the methods of the present invention follow Scheme III described below.

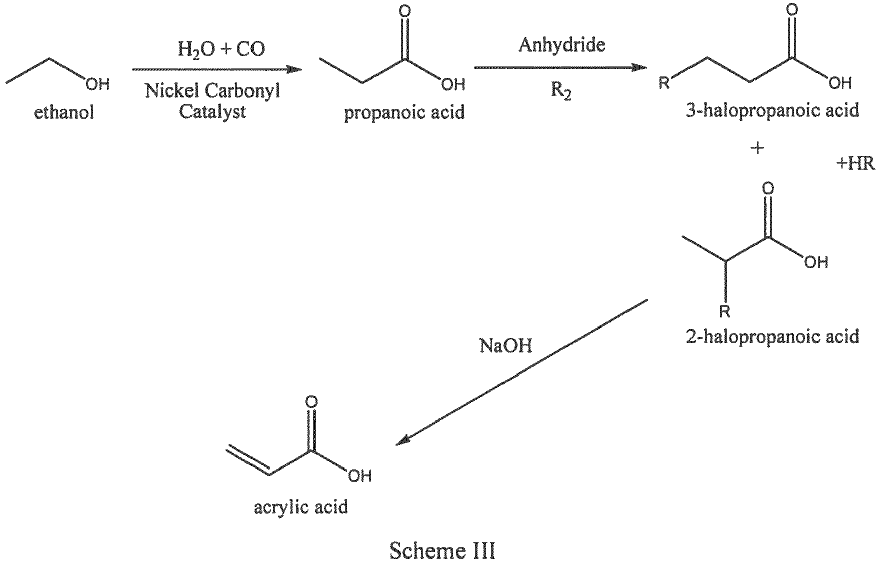

Wherein R is a halogen atom selected from the group consisting of fluorine, chlorine, bromine, and iodine.

In some embodiments, the methods of the present invention comprise the following steps:
(e) providing a form of ethanol derived from a fermentation process;
(f) carbonylating the ethanol in the presence of a metal catalyst to obtain propanoic acid;
(g) chlorinating the propanoic acid to obtain a mixture comprising 3-chloropropanoic and 2-chloropropanoic acid; and
(h) heating the mixture comprising 3-chloropropanoic and 2-chloropropanoic acid in the presence of an inorganic base to obtain acrylic acid.

In some embodiments, step (g) is carried out in the presence of an anhydride (e.g. acetic anhydride).

In some embodiments, the methods of the present invention follow Scheme IV described below.

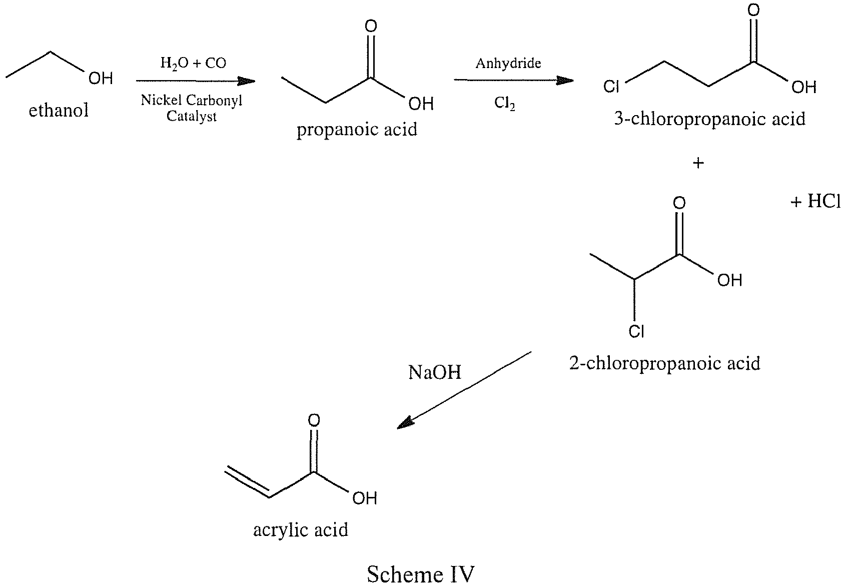

The invention will be described in greater detail by way of specific examples. The following examples are offered for illustrative purposes and are not intended to limit the invention in any manner. Those skilled in the art will readily recognize a variety of noncritical parameters which can be changed or modified to yield essentially the same results.

### Example

### Example 1

Fifty (50) grams of 3-chloropropanoic acid is added to a three-neck round bottom flask and heated until it reaches a temperature of 60 °C. Thirty-one (31) grams of potassium hydroxide is added to a beaker containing fifty (50) mL of water and the mixture is stirred until the potassium hydroxide dissolves. The potassium hydroxide dilution is slowly added to the three-neck round bottom flask containing 3-chloropropanoic acid so that the temperature never exceeds 80 °C. The mixture of potassium hydroxide and 3-chloropropanoic acid is stirred for about 1 hour. Phosphoric acid is slowly added to the reaction mixture to acidify the solution. The heat is gently increased until the water distills off around 100 °C, and the water is collected. 0.005 grams of 4-methoxyphenol is added to a new flask for collecting acrylic acid. The contents of the new flask are gently heated until acrylic acid distills off around 139 °C and the acrylic acid is collected.

It is intended that any patents, patent applications or printed publications, including books, mentioned in this patent document be hereby incorporated by reference in their entirety.

As those skilled in the art will appreciate, numerous changes and modifications may be made to the embodiments described herein, without departing from the spirit of the invention. It is intended that all such variations fall within the scope of the invention.

## Claims

1. A method for preparing acrylic acid comprising the followings steps:
(a) providing a source of ethanol;
(b) carbonylating the ethanol in the presence of a metal catalyst to obtain propanoic acid;
(c) halogenating the propanoic acid to obtain halopropanoic acid;
(d) heating the halopropanoic acid in the presence of an inorganic base; and
(e) recovering acrylic acid.

2. The method of claim 1, wherein the metal catalyst is a metal carbonyl catalyst.

3. The method of claim 1 or claim 2, wherein the metal catalyst is a nickel carbonyl catalyst.

4. The method of any foregoing claim, wherein the halopropanoic acid is chloropropanoic acid.

5. The method of claim 4, wherein the chloropropanoic acid product of step (c) comprises a mixture of 3-chloropropanoic acid and 2-chloropropanoic acid.

6. The method of any foregoing claim, wherein the inorganic base is potassium hydroxide.

7. The method of any foregoing claim, wherein the halopropanoic acid is heated to a temperature that does not exceed about 80 °C.

8. The method of claim 8, wherein the inorganic base is added to the halopropanoic acid at a rate sufficient to maintain the temperature of the reaction mixture at 80 °C or below.

9. The method of any foregoing claim, wherein the method further comprises acidifying the solution of halopropanoic acid and inorganic base.

10. The method of claim 9, wherein the solution of halopropanoic acid and inorganic base is acidified with an inorganic acid, optionally wherein the inorganic acid is phosphoric acid.

11. The method of claim 9 or claim 10, wherein the acidified solution is heated to a temperature of about 100 °C to distill off the water.

12. The method of any foregoing claim, wherein the acrylic acid is recovered through distillation, optionally wherein the acrylic acid has a purity of 90% or greater.

13. The method of claim 1, further comprising the step of esterifying the acrylic acid to obtain an acrylate ester.

14. A coating for a substrate comprising a product of the method of any foregoing claim.

15. The coating of claim 14, wherein the substrate is a flooring product.
